# EUROPÄISCHE PATENTSCHRIFT

(11) **EP 1 986 582 B1**
(45) Veröffentlichungstag und Bekanntmachung des Hinweises auf die Patenterteilung: **26.09.2012**
(21) Anmeldenummer: 07711596.2
(22) Anmeldetag: 20.02.2007
(51) Int. Cl.: A61F 9/01, B23K 26/04

(54) **VORRICHTUNG UND VERFAHREN ZUM DETEKTIEREN DER FOKUSLAGE EINES OPTISCHEN SYSTEMS UND OPHTHALMOLOGISCHE BEHANDLUNGSVORRICHTUNG**
APPARATUS AND METHOD FOR THE DETECTION OF THE FOCUSED POSITION OF AN OPTICAL SYSTEM, AND OPHTHALMOLOGICAL TREATMENT APPARATUS
DISPOSITIF ET PROCEDE DE DETECTION DE LA POSITION FOCALE D'UN SYSTEME OPTIQUE, ET DISPOSITIF DE TRAITEMENT OPHTALMOLOGIQUE

(30) Priorität: 20.02.2006 DE 102006007750
(43) Veröffentlichungstag der Anmeldung: 05.11.2008
(73) Patentinhaber: WaveLight GmbH, 91058 Erlangen (DE)
(72) Erfinder: KITTELMANN, Olaf, 14163 Berlin (DE); TRIEBEL, Peter, 07751 Jena (DE)
(74) Vertreter: von Hellfeld, Axel
(86) Internationale Anmeldenummer: PCT/EP2007/001456
(87) Internationale Veröffentlichungsnummer: WO 2007/096136

(56) Entgegenhaltungen:
- WO-A-2005/039462
- DE-A1- 10 356 415
- US-A- 5 676 866
- US-A1- 2004 051 976
- US-B1- 6 448 534

## Beschreibung

Die Erfindung betrifft eine Vorrichtung und ein Verfahren zum Detektieren der Fokuslage eines optischen Systems. Insbesondere betrifft die Erfindung eine Vorrichtung und ein Verfahren zum Detektieren der Fokustiefe eines abbildenden optischen Systems und darüber hinaus auch eine Vorrichtung und ein Verfahren zum Steuern der Fokuslage und insbesondere Fokustiefe. Weiterhin betrifft die Erfindung auch eine ophthalmologische Behandlungs- und/oder Diagnosevorrichtung unter Verwendung der genannten Vorrichtung bzw. des genannten Verfahrens.

Bei den hier in Rede stehenden optischen Systemen handelt es sich insbesondere um ein abbildendes optisches System in einer Materialbearbeitungsanlage unter Verwendung von Lichtquellen, wie insbesondere Lasern und LEDs. Unter Materialbearbeitung ist hier auch eine Materialstrukturierung im Mikrobereich zu verstehen, z. B. für dielektrische Materialien, wie z. B. biologisches Gewebe, oder auch metallische Materialien. Insbesondere kann die Erfindung Anwendung finden bei ophthalmologischen optischen Systemen, insbesondere in der refraktiven Hornhautchirurgie, wie z. B. LASIK. Dabei ist ein besonders für die vorliegende Erfindung geeigneter Anwendungsbereich die fs-LASIK, also die refraktive Homhautchirurgie unter Einsatz eines Femtosekundenlasers.

Bei den vorstehend genannten optischen Abbildungssystemen kommt es für die Erzielung hochpräziser Materialbearbeitungen unter anderem auf eine genaue Kontrolle der Fokuslage an. Unter "Fokuslage" wird hier von allem aber nicht nur der Ort des Fokus in Richtung der optischen Achse (die sogenannte Fokustiefe) verstanden, sondern allgemeiner auch die Position und Orientierung der fokussierten Strahlung, also z. B. ein Versatz in Bezug auf die ideale optische Achse des Systems oder eine Winkelstellung der tatsächlichen Achse der optischen Strahlung in Bezug auf die ideale (gewünschte) optische Achse. Bei der fs-LASIK kommt es ganz besonders auf die Einhaltung der berechneten Fokustiefe an und dies ist ein besonderer Einsatz der vorliegenden Erfindung.

In der WO 2005/039462 wird mittels am Kontaktglas angeordneter Reflektorzonen die Lage der Augenhornhaut bestimmt, um in der nachfolgenden Behandlung den Fokus relativ zu dieses ist-lage zu plazieren.

In DE 10 2004 009 212 A1 wird ein optisches Köntaktelement zur Lasermaterialbearbeitung vorgestellt. Dieses Kontaktelement wird in der bevorzugten Ausführungsform für fs-LASIK verwendet. Dabei besteht dieses Kontaktelement aus einer diffraktivoptischen Struktur. Diese Strukturen sollen die durch hohe numerische Aperturen des Objektivs auftretenden Einfallswinkel minimieren. Das diffraktiv optische Element (DOE) besteht dabei aus einer Gitterstruktur mit radial-angepasster Gitterperiode. Die Gitterperioden liegen dabei zwischen 200l/mm bis 500l/mm. Als Spotgrößen werden Werte im µm-Bereich angegeben. Aufgrund optischer Begrenzungen ist nur eine numerische Apertur von ca. 0.3 möglich. Eine Vergrößerung der Apertur wird durch das Einsetzen eines zweiten diffraktiven Elements in den Strahlengang des Objektives erreicht. Dieses DOE ist ebenfalls als zirkuläre Gitterstruktur mit einer zur optischen Achse größer werdenden Gitterperiode ausgeführt. Als Vorteil dieser Ausführung wird dabei die Erzielung von höheren numerischen Aperturen angegeben. Das Kontaktelement ist weiterhin gekrümmt ausgeführt. Der Krümmungsradius entspricht dabei dem Krümmungsradius des Auges, ca. 8 mm. Die Materialbearbeitung wird mit diesem einheitlich voreingestellten Krümmungsradius durchgeführt. Die Ansaugung wird ähnlich WO 03/002008 A1 und EP 1 159 986 A2 durchgeführt. Eine Fokuskontrolle wird mit diesem vorgestellten Verfahren nicht durchgeführt.

In der EP 0 627 675 A1 wird eine diffraktiv optische Einrichtung zur Abbildung eines oder mehrer Raumpunkte aus einem Strahl vorgestellt. Dabei besteht die diffraktive Struktur ebenfalls aus einer segmentartigen Anordnung von beliebigen binären oder mehrstufigen diffraktiven Elementen. Die Anordnung kann insbesondere eine hexagonale oder sechseckige Anordnung sein. Damit wird eine Abbildung eines Lichtstrahles erreicht. Es wird jedoch lediglich eine Intensitäts- oder/und Phasentransformation vorgenommen.

In der US 2002/0171028 ist eine Vorrichtung zur Fokuskontrolle beschrieben. Dabei wird das zurückrufende Licht durch einen Abbiidungsstrahlengang zur Interferenz mit einem zweiten Strahlenbündel gebracht und somit eine interferometrische Wellenkontrolle durchgeführt.

Ebenso wird in der US 6,666,857 B2 eine Fokuskontrolle mittels einer interferometrischen Wellenfrontkontrolle durchgeführt. Die aktive Wellenfrontsteuerung während des Photoablationsprozesses am menschlichen Auge wird dann durch eine Kombination von adaptiven Spiegeln erreicht. Es soll keine aktive Wellenfrontsteuerung vorgenommen werden.

In der US 2004/0051976 A1 wird eine optische Anordnung eines konfokalen Mikroskops, bestehend aus einer Laserquelle, vorwiegend emittierend im UV-Spektralbereich, einer Strahlaufweitungsoptik, einem diffraktivem Pinhole-Array und einem Objektiv beschrieben. Ein diffraktives Pinhole-Array wird in seiner genauen Ausführungsform nicht beschrieben. Als ein Vorteil dieser technischen Ausführungsform kann die Effizienzsteigerung gesehen werden, so haben Amplituden-Pinhole Arrays, in Abhängigkeit des Öffnungsverhältnisses eine typische Transmission zwischen 4% und 10%. Mit einem diffraktiven Pinhole Array sind hingegen Transmissionswerte eines solchen optischen Elementes von bis zu 80% möglich, eine Abhängigkeit vom Öffnungsverhältnis oder aber der Anzahl der Pinholes ist dabei nur herstellungsbedingt gegeben.

In der US 2004/0021851 wird eine optische Anordnung bestehend aus einem Laser und nachfolgender Strahlformungsoptik zur Vermessung der Brennweite einer unbekannten Linse verwendet. Die Messung der Brennwerte wird dabei durch das Fokussieren auf eine Referenzoberfläche mit verschiedenen Abständen durchgeführt. Es wird dabei der rückreflektierte Anteil der Strahlung detektiert. Die Spotdurchmesser werden dann mit den jeweiligen Abständen ausgewertet. Mittels der "Newtonschen" Relation Z Z'=f² wird die Brennweite bestimmt. Ein optisches Gitter, welches nicht näher beschrieben ist, wird zur Auskopplung des rückreflektierten Anteils der Strahlung verwendet. Der Jones-Matrix Formalismus wird ebenfalls zur Berechnung der Brennweite herangezogen. Die Genauigkeit des Verfahrens liegt bei 1%.

In der US 6,909,546 B2 wird eine optische Anordnung bestehend aus einer Lichtquelle (Nd:YAG2w) und nachfolgender Strahlformungsoptik beschrieben. Dabei werden zwei diffraktiv-optische Elemente eingesetzt, um die Laserstrahlung zu homogenisieren. Das erste der beiden DOE wird dabei zur Homogenisierung und Raumfrequenzfilterung verwendet. Ein nachfolgendes Pinhole führt die Raumfrequenzfilterung durch. Innerhalb des 2f-Aufbaus der Raumfrequenzfilterung befindet sich das zweite DOE, welches die gewünschte Intensitätsverteilung im Fernfeld erzeugt. Das Fernfeld wird entweder durch die Feldlinse oder aber das 2. DOE erzeugt. Im Fokus wird die gewünschte Intensitätsverteilung erzeugt. Eine Fokuskontrolle wird bei diesen Verfahren nicht durchgeführt.

Die Erfindung hat dementsprechend das Ziel, eine Vorrichtung und ein Verfahren bereitzustellen, mit denen die Fokuslage eines optischen Systems genau feststellbar ist.

Hierzu stellt die Erfindung eine Vorrichtung bereit zum Detektieren der Fokuslage eines optischen Systems mit einer Strahlungsquelle, einem fokussierenden Abbildungssystem, einer zumindest teilreflektierenden Fläche am Fokus, einem geeigneten digitalen Sensorsystem (z. B. CCD-Kamera, CMOS-Kamera oder dergleichen) zur Aufnahme eines von der genannten Fläche reflektierten Bildes, einem Rechner zum Auswerten des von der Kamera aufgenommenen Bildes, und mit einem optischen Element im Strahlengang des optischen Systems vor dem fokussierenden Abbildungssystem, welches das genannte Bild in Abhängigkeit von der Fokuslage beeinflusst.

Dabei ist das genannte fokussierende optische Abbildungssystem bevorzugt eine Fokussierungsoptik mit einstellbarer (variabler) Fokuslage, insbesondere also ein System, mit dem der Ort des Fokus in einer Richtung parallel zur optischen Achse der Abbildung (also die Fokustiefe) einstellbar ist. Darüber hinaus ist bei einem solchen System in der Regel auch die Fokuslage in einer Richtung senkrecht zur optischen Achse der Strahlung einstellbar, z. B. bei fs-LASIK.

Die erfindungsgemäße Vorrichtung und das entsprechende Verfahren dienen also insbesondere zur anfänglichen Einstellung und Justierung eines optischen Systems derart, dass unmittelbar vor der Materialbearbeitung in Bezug auf eine vorgegebene Ebene, die sogenannte Fläche, der Fokus genau eingestellt wird, insbesondere so, dass er genau auf dieser Fläche liegt. Bei Einsatz in der LASIK ist die genannte Nullebene bevorzugt eine Fläche, die dadurch entsteht, dass die Komea im interessierenden Bereich an eine Reverenzfläche angesaugt wird (dies ist als solches dem LASIK-Fachmann bekannt). Die flachdrückende, für die verwendete Strahlung transparente Scheibe ist auf ihrer der Komea zugekehrten und an dieser anliegenden Seite so beschichtet, dass die einfallende Strahlung zu einem geringen Prozentsatz reflektiert wird. Diese Reflexion erzeugt dann das genannte Bild der auf dieser Nullebene fokussierten Strahlung, weiches mit der genannten Kamera vermessen und ausgewertet wird. Bei idealer Fokussierung soll also der Fokus genau auf dieser Nullebene (im dargestellten Beispiel also im wesentlichen auf der flachgedrückten Korneaoberfläche) liegen und entsprechend der Auswertung des reflektierten Bildes wird dann das optische System so eingestellt, dass die Fokussierung optimal ist, also die Lage des Fokus genau in dieser Nullebene ist. Damit ist das optische System eingestellt und justiert und kann für die nachfolgende Materialbearbeitung eingesetzt werden. Bei der nachfolgenden Materialbearbeitung wird in der Regel die Lage des Fokus in Bezug auf die genannte Nullebene verändert. So wird z. B. bei der fs-LASIK beim Schneiden des sogenannten Flap der Fokus in das Stroma gelegt und es werden sukzessive quer zur optischen Achse die Fokus-Positionen variiert, um das Flap zu erzeugen. Dies ist als solches bekannt. Die vorstehend beschriebene anfängliche Einstellung des Systems gewährleistet eine genaue Positionierung der Foci an den gewünschten Soll-Stellen.

Bei anderen Materialbearbeitungen kann die Nullebene, die auch als Referenzebene bezeichnet werden kann, anders definiert werden und muss nicht notwendig mit der Oberfläche des zu bearbeitenden Materials zusammenfallen. Die auf die Nullebene fokussierte Strahlung und die Vermessung des in dieser Ebene reflektierten Bildes liefert eine Eichung des optischen Systems derart, dass die Einstellung der optischen Abbildungseigenschaften des optischen Systems für den Idealzustand der Fokussierung genau in der Nullebene aufgrund der Bildvermessung bekannt ist, so dass anschließend, von diesen Einstellungen des optischen Systems ausgehend, die Fokuslage entsprechend der gewünschten Materialbearbeitung, z.B. in das innere der Kornea, verändert werden kann.

Gemäß einer Ausgestaltung ist das genannte optische Element, welches das zu vermessende Fokusbild in Abhängigkeit von der Fokuslage beeinflusst, eine Blendenmatrix (sogenanntes pin hole array).

Bei dem optischen Element kann es sich auch um ein sogenanntes diffraktives optisches Element (DOE) handeln, welches ein Punktmuster in der Fernfeldverteilung erzeugt (als solches dem Fachmann bekannt und hier nicht näher erläutert).

Das genannte optische Element kann im Strahlengang des reflektierten Bildes zwischen der reflektierenden Fläche und der Kamera angeordnet sein, oder auch außerhalb dieses Strahlenganges, je nach Einsatzart ergeben sich jeweils Vorteile.

Mit dem optischen Element kann bevorzugt die Amplitude (Intensität) oder die Phase (Wellenfront) des reflektierten Bildes lokal beeinflusst werden und es können die Defokusanteile der Wellenfront sichtbar gemacht werden.

Es ist auch möglich, das genannte optische Element im Strahlengang sowohl phasenempfindlich als auch amplitudenempfindlich vorzusehen, insbesondere eine Kombination davon.

Gemäß einer bevorzugten Ausgestaltung erzeugt das optische Element ein Punktmuster, insbesondere ein regelmäßiges matrixförmiges Punktmuster.

Die Erfindung stellt auch ein Verfahren bereit zum Detektieren der Fokuslage eines optischen Systems, bei dem die Strahlung einer Strahlungsquelle über ein fokussierendes Abbildungssystem in eine Fokusebene abgebildet wird und wobei zur Feststellung der Fokuslage des optischen Systems unter Einschluss des abbildenden Systems ein Bild am Fokus erzeugt wird, welches dort reflektiert wird und mit einer Kamera aufgenommen wird, wobei ein optisches Element das aufgenommene Bild in Abhängigkeit von der Fokussierung der Strahlung beeinflusst und in Abhängigkeit von der genannten Beeinflussung des Bildes eine Information über die Fokuslage der fokussierten Strahlung am vorgesehenen Fokusort abgeleitet wird.

Nachfolgend werden Ausführungsbeispiele der Erfindung anhand der Zeichnung näher beschrieben. Es zeigt bzw. zeigen:
Fig. 1 schematisch ein erstes Ausführungsbeispiel eines optischen Systems mit einer Vorrichtung zum Detektieren einer Fokuslage;
Fig. 2 ein zweites Ausführungsbeispiel eines optischen Systems mit einer Vorrichtung zum Detektieren der Fokuslage;
Fig. 3 schematisch ein Ausführungsbeispiel einer Anordnung gemäß Fig. 2 mit schematischer Darstellung von Phasenverteilungen der Strahlung im System und mit einer Lochmatrix;
Fig. 4 ein Ausführungsbeispiel einer Anordnung gemäß Figur 2 mit einem diffraktiven optischen Element; und
Fig. 5, 6 Ausführungsbeispiele für mit einer Kamera aufgenommene Bilder bei einer fokussierenden Abbildung in der Art einer Lochmatrix mit genauer Fokussierung bzw. Fokussierfehlem.

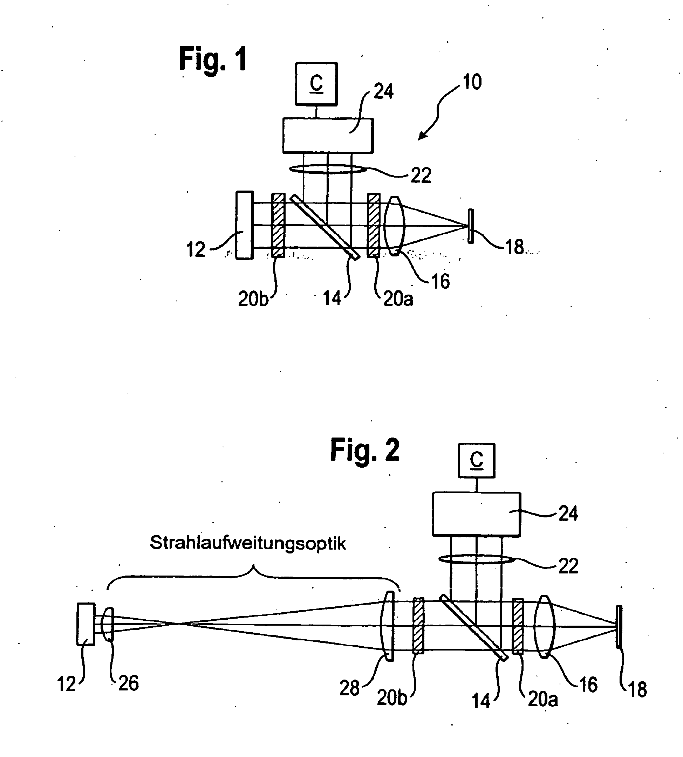

Entsprechend Fig. 1 weist ein optisches System 10 eine Lichtquelle 12 auf, die z. B. ein Laser (wie z. B. ein fs-Laser) oder eine LED etc. sein kann. Die von der Lichtquelle 12 abgegebene Strahlung durchläuft einen Auskoppelspiegel 14 und wird über ein fokussierendes Abbildungssystem 16 auf eine Ebene 18 fokussiert. Das fokussierende Abbildungssystem 16 ist in den Figuren nur schematisch durch eine einzige Linse angedeutet. Üblicherweise weist das fokussierende Abbildungssystem 16 eine Mehrzahl von Linsen auf, von denen eine oder mehrere zur Einstellung und Änderung des Fokus betätigbar sind. Solche optischen Abbildungssysteme sind als solches bekannt.

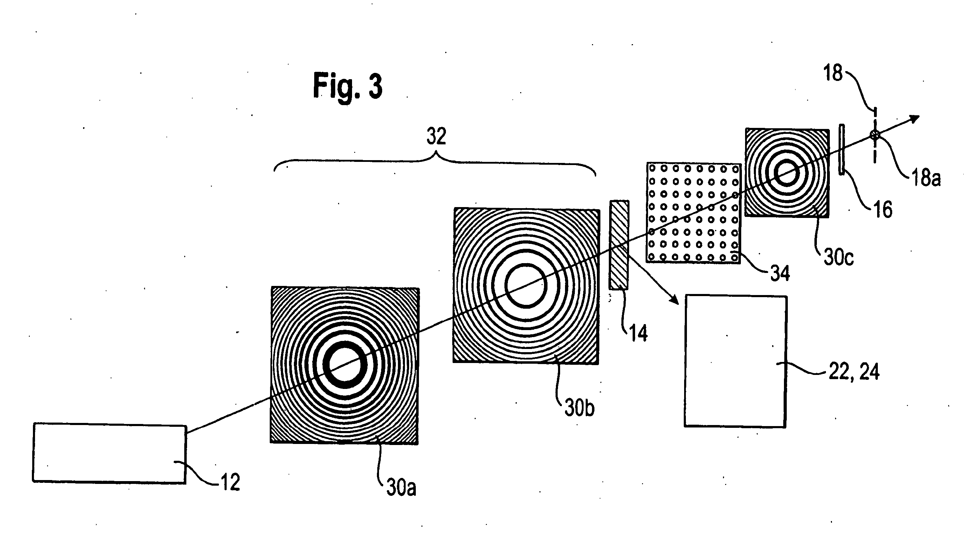

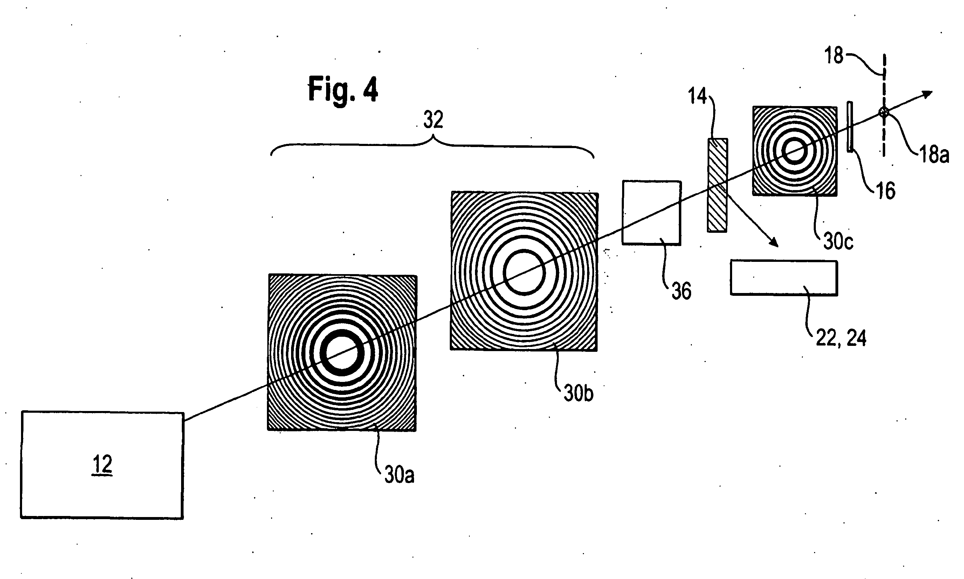

In Fig. 1 sind mit den Bezugszeichen 20a und 20b Bereiche (Orte) markiert, an denen wahlweise ein unten näher beschriebenes optisches Element zu positionieren ist. Beispiele derartiger optischer Elemente sind die in den Figuren 3 und 4 gezeigten optischen Elemente 34 bzw. 36.

Von der reflektierenden Fläche 18 reflektierte Strahlung gelangt über das optische Abbildungssystem 16 und gegebenenfalls das im Bereich 20a angeordnete, weiter unten näher beschriebene optische Element auf den Auskoppelspiegel 14 und wird von dort in Fig. 1 nach oben über eine Abbildungsoptik 22 auf eine digitale Kamera 24 abgelenkt, z.B. eine sogenannte CCD-Kamera mit hoher örtlicher Auflösüng. Das mit der Kamera 24 aufgenommene digitale Bild wird in einen Rechner C eingegeben und dort ausgewertet, wie weiter unten näher beschrieben ist.

Fig. 2 zeigt ein abgewandeltes Ausführungsbeispiel, wobei in den Figuren funktionsgleiche oder funktionsähnliche Bauteile und Merkmale mit den gleichen Bezugszeichen versehen sind. Beim Beispiel gemäß Fig. 2 ist eine Strahlaufweitungsoptik (Teleskop) bestehend aus den optischen Elementen 26, 28 vorgesehen, um den Strahl vor seiner Fokussierung mit dem Abbildungssystem 16 aufzuweiten. Statt des in der Figur dargestellten Keppler-Teleskopes kann auch ein anderes Strahlformungssystem an dessen Stelle eingesetzt werden. Allgemein kann das in Figur 2 mit "Strahlaufweitungsoptik" bezeichnete optische System auch eine Strahlformungsoptik sein.

Wie oben bereits erwähnt, kann in den Bereichen 20a und/oder 20b gemäß den Figuren 1 und 2 ein optisches Element angeordnet werden, welches in Abhängigkeit von der mehr oder weniger optimalen Fokussierung mittels des Abbildungssystems 16 auf die reflektierende Fläche 18 das oben beschriebene, durch Reflexion entstehende und mit der Kamera 24 aufgenommene Bild beeinflusst und so eine Feststellung darüber ermöglicht, ob die Fokussierung auf die der Fläche 18 entsprechende Ebene genau die gewünschte ist oder ob die Fokuslage in Bezug auf diese Ebene verschoben ist, z. B. in Richtung der optischen Achse zu weit vorne liegt oder zu weit hinten (sogenannte Fokustiefe).

Gemäß Fig. 3 ist als optisches Element im vorstehenden Sinne eine Lochmaske 34 im Strahlengang vor dem fokussierenden Abbildungssystem 16 angeordnet.

Im Idealfall ist also das optische Abbildungssystem 16 so eingestellt, dass die von der Lichtquelle 12 kommende Strahlung genau in der Ebene 18 an einer vorgegebenen Stelle fokussiert ist. Der Fokus ist in Fig. 3 mit dem Bezugszeichen 18a markiert. Das Ausführungsbeispiel gemäß Fig. 3 entspricht dem Beispiel entsprechend Fig. 2 mit einer Strahlaufweitungsoptik im Bereich, der mit dem Bezugszeichen 32 angedeutet ist. Dort sind auch die Phasenverteilungen durch die Bezugszeichen 30a, 30b, 30c symbolisch markiert.

Das optische Element 34 ist eine Lochmatrix mit N x M einzelnen Löchern in der dargestellten regelmäßigen Anordnung. Das optische Element kann bei diesem Ausführungsbeispiel als reines amplitudenbezogenes Element ausgefürt sein, also Intensitäten der Strahlung beeinflussen. Typische Lochdurchmesser in der Lochmaske liegen zwischen 1 µm und 100 µm. Die Löcher können insbesondere sechseckig, quadratisch, hexagonal oder auch kreisförmig angeordnet sein. Die Anordnung der einzelnen Löcher richtet sich nach dem verwendeten Strahlprofil und den Anforderungen an die Genauigkeit hinsichtlich der Fokuslage. Mit dem beschriebenen System können Fokuslagen bis auf wenige µm genau bestimmt werden. Da die Strahlung auf dem Weg zur Ebene 18 und das in der Ebene 18 reflektierte Bild jeweils durch das optische Element 34 laufen, erfolgt eine Beeinflussung des mit der Kamera 24 gemessenen Bildes in Abhängigkeit von der Genauigkeit der Fokussierung in der Ebene 18. Eine Änderung der Fokuslage in Bezug auf die Ebene 18 (welche die oben definierte Nullebene ist) von wenigen Mikrometern kann durch Auswertung des mit der Kamera 24 aufgenommenen Bildes im Rechner C detektiert werden.

Es ist auch möglich, die im Fokus auftretende Strahlungsleistung durch Integration der mit der Kamera 24 an den einzelnen Bildpunkten gemessenen Intensitäten zu bestimmen.

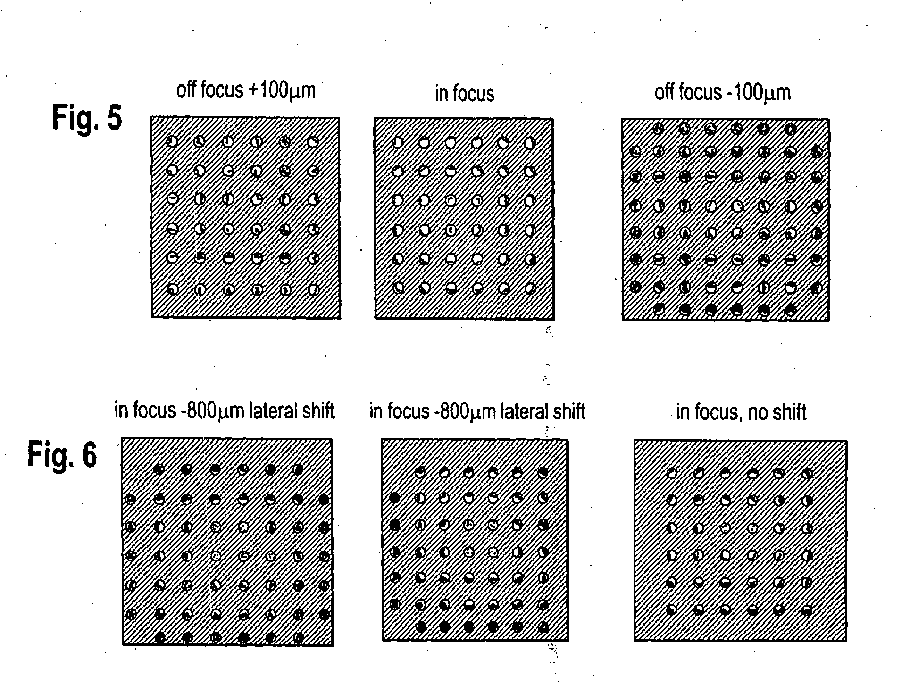

Fig. 5 zeigt beispielhaft und schematisch auf diese Weise gewonnene und ausgewertete Reflexionsbilder. Dabei zeigt Fig. 5 in der Mitte das gewonnene, matrixartige Lochbild für den Fall, dass das optische System einschließlich des fokussierenden Abbildungssystems 16 so eingestellt ist, dass die Fokussierung exakt am gewünschten Ort in der Nullebene 18 liegt. Wie gesagt, liegt die reflektierende Fläche zur Erzeugung des vermessenen Bildes auch in dieser Ebene 18. Wie das Lochbild in Fig. 5, Mitte, zeigt, sind im reflektierten Bild die einzelnen Löcher ohne sphärischen Anteil vollständig homogen, entsprechend dem Eingangsstrahlprofil ausgeleuchtet.

Fig. 5 zeigt im linken Lochbild eine Verschiebung der Fokuslage in Bezug auf die Nullebene 18 nach hinten um ca. 100 µm. Die Bildauswertung ergibt im Vergleich zur exakten Fokussierung (Fig. 5, Mitte) eine Abänderung der einzelnen Bildpunkte in der Matrix und der Rechner C ist für die Auswertung so kalibriert, dass er diese Abweichung "erkennt". Die Kalibrierung des Rechners kann z. B. experimentell derart erfolgen, dass mit einem bekannten optischen Abbildungssystem gezielt Änderungen des erzeugten reflektierten Bildes in Abhängigkeit von der Fokuslage aufgenommen und abgespeichert werden, so dass anschließend durch Vergleich mit tatsächlich gemessenen Bildern die Fokuslage ermittelt werden kann.

Fig. 5 zeigt rechts eine Defokussierung um-10 µm mit einer Objektivbrennweite von 50 mm mit einer entsprechenden Abänderung des Lochbildes im Vergleich zur idealen Fokussierung. Allgemein gesprochen erlaubt die Asymmetrie des Bildes, wie es in Fig. 5 links bzw. rechts gezeigt ist, eine Analyse der Fokussierung. Ergibt diese Analyse aufgrund der Bildauswertung mit dem Rechner C eine asymmetrische Helligkeitsverteilung im Bild, dann können Elemente des fokussierenden Abbildungssystems 16 so lange verändert werden, bis die Bildauswertung ergibt, dass der Fokus exakt in der Ebene 18 liegt.

Fig. 4 zeigt ein Ausführungsbeispiel der Vorrichtung zum Detektieren der Fokuslage eines optischen Systems 10, bei dem das optische Element beim Ausführungsbeispiel nach Fig.2 im Bereich 20b angeordnet ist, also derart, dass das an der Ebene 18 reflektierte Bild auf dem Weg zur Kamera 24 nicht durch das optische Element 36 läuft.

Das optische Element 36 ist in diesem Falle ein diffraktives optisches Element (DOE), welches z. B. einen "1 auf N" Strahlteiler bildet, also einen einfallenden Einzelstrahl in N Einzelstrahlen aufteilt, wobei N z. B. zwischen 2 und 50 variieren kann. Die durch das diffraktive optische Element 36 verursachte Divergenz kann durch eine zweite (nicht gezeigte) Struktur refraktiv oder diffraktiv korrigiert werden. Es können auch mehrere diffraktive optische Elemente hintereinander angeordnet werden, je nach Strahlprofil und gewünschter Analyse. Ein Vorteil einer Anordnung mit diffraktiven optischen Elementen ist die Möglichkeit der Korrektur der einfallenden Phasenverteilung. Die Phasenverteilung kann sowohl durch die Lichtquelle als auch die nachfolgenden optischen Elemente, also insbesondere die Strahlaufweitungsoptik, beeinflusst werden. Auch bei diesem Ausführungsbeispiel wird, analog der Beschreibung anhand der Fig. 3, das in der Ebene 18 reflektierte Bild mit der Kamera 24 aufgenommen und im Rechner C ausgewertet. Fig. 6 zeigt für den Fall, dass das diffraktive optische Element eine matrixartige Strahlungsverteilung erzeugt, drei mit der Kamera 24 aufgenommene Bilder, wobei das Bild rechts in Fig. 6 den Fall einer idealen Fokussierung anzeigt mit einer relativ gleichmäßigen Ausleuchtung der einzelnen Bildpunkte. In Fig. 6 links ist der Fall dargestellt, dass die Fokuslage seitwärts vom idealen Abbildungspunkt 18a abweicht, und zwar um einige hundert Mikrometer. Die einzelnen bildpunkte sind asymmetrisch ausgeleuchtet. Fig. 6 zeigt in der Mitte den Fall einer in anderer Richtung seitwärts verschobenen Fokuslage, wobei ebenfalls die einzelnen matrixartigen Lichtpunkte weniger symmetrisch ausgeleuchtet sind wie im Falle der idealen Fokussierung gemäß dem Bild in Fig. 6, rechts.

Ein optisches Element 36 in Form eines DOE hat gegenüber einer Lochmatrix den Vorteil einer hohen Transmission. Mit einem diffraktiven Element kann typischerweise eine Effizienz zwischen 80 und 90 % erreicht werden. Auch ermöglicht eine solche Anordnung eine sehr hohe Dynamik bei der Auswertung der Fokuslage, d. h. Abweichungen des Fokus von der idealen Solllage können über einen weiten Bereich festgestellt werden.

Es ist auch möglich, das diffraktive optische Element 36 in den Bereichen 20a gemäß den Figuren 1 und 2 anzuordnen.

Auch kann das diffraktive optische Element als binäres Element oder auch als sogenannte Multi-Level-Gitterstruktur ausgeführt werden. Die Gitterstrukturen können eindimensional oder auch zweidimensional sein.

Wird eine Anordnung gemäß den Figuren 1, 2, 3 oder 4 in der fs-LASIK eingesetzt, dann kann es sich bei der reflektierenden Fläche 18, welche die oben erläuterte Nullebene definiert, z. B. um die Rückseite eines transparenten Plättchens in einer als solches bekannten Ansaugvorrichtung handeln, die so beschaffen ist (beschichtet oder unbeschichtet), dass ein geringer Prozentsatz der einfallenden Strahlung zur Gewinnung des mit der Kamera 24 aufzunehmenden Bildes reflektiert wird.

Als diffraktive optische Elemente werden insbesondere eingesetzt: Gitter, Fresnel-Zonen-Linsen, sogenannte Beam-Shaping-Elemente, etc. Auch können als Element (36) sogenannte refraktiv-optische Komponenten eingesetzt werden: z. B. Mikrolinsenarrays, Beam-Shaping-Elemente, etc. Dient das optische Element 34 der Amplitudenanalyse, dann eignen sich insbesondere Lochmasken oder auch Anordnungen von Löchern in beliebiger Geometrie wie quadratisch, sechseckig, hexagonal, etc., je nach Strahlart und Analyseziel.

Auch kann das optische Element als Spalt ausgebildet sein oder als Anordnung mehrerer Spalte.

Mit den beschriebenen Anordnungen kann nicht nur die Fokuslage bestimmt und gesteuert werden, sondern es können auch Strahldivergenzen, Laserleistungen, Abweichungen der Strahlung von der optischen Achse, Abweichungen im sogenannten Strahlprodukt M² oder auch Änderungen des Ausgangsstrahlprofils der Lichtquelle 12 erfasst werden, da alle diese Strahlparameter Einfluss haben können auf das mit der Kamera 24 aufgenommene reflektierte Bild. Hinsichtlich all dieser Strahlparameter kann der Rechner C zuvor experimentell durch gezielte Versuche mit einer Datenbank versehen werden, welche einzelnen Strahlparametern Abweichungen von den idealen Sollwerten zuordnet, die jeweils Bildänderungen entsprechen, so dass das System durch Eingriff mit entsprechenden Stellgrößen auf ideale Werte justierbar ist. Dabei ermöglicht der Einsatz von diffraktiv optischen Elementen eine Kompensation von möglicherweise auftretenden Phasenänderungen im Strahlengang, welche auch die Fokuslage beeinflussen können. Eine solche Analyse ermöglicht der als solches bekannte Hartmann-Shack-Sensor nicht.

## Patentansprüche

1. Vorrichtung zum Detektieren der Fokuslage eines optischen Systems (10) einer LASIK-Anordnung mit einer Strahlungsquelle (12), einem fokussierenden Abbildungssystem (16), einer zumindest teilreflektierenden Fläche (18), die an der Rückseite eines transparenten Plättchens einer Ansaugvorrichtung ausgebildet ist, einem digitalen Sensorsystem (24) zur Aufnahme eines von der genannten Fläche (18) reflektierten Bildes, einem Rechner (C) zum Auswerten des von dem digitalen Sensorsystem (24) aufgenommenen Bildes, und mit einem optischen Element (34; 36) im Strahlengang des optischen Systems (10) vor dem fokussierenden Abbildungssystem (16), **dadurch gekennzeichnet, dass** das optische Element (34; 36) im Strahlengang die Phase oder Amplitude des genannten Bildes in Abhängigkeit von der Fokuslage beeinflusst, wobei die teilreflektierende Fläche (18) einen Prozentsatz der einfallenden Strahlung zur Gewinnung des mit dem digitalen Sensorsystem aufzunehmenden Bildes reflektiert.

2. Vorrichtung nach Anspruch 1, **dadurch gekennzeichnet, dass** das optische Element (34; 36) eine Lochmatrix ist.

3. Vorrichtung nach Anspruch 1, **dadurch gekennzeichnet, dass** das optische Element (34; 36) ein diffraktiv optisches Element ist.

4. Vorrichtung nach einem der vorhergehenden Ansprüche, **dadurch gekennzeichnet, dass** das optische Element (34) im Strahlengang des genannten reflektierten Bildes angeordnet ist.

5. Vorrichtung nach einem der Ansprüche 1 bis 3, **dadurch gekennzeichnet, dass** das optische Element (36) außerhalb des Strahlenganges des reflektierten Bildes angeordnet ist.

6. Vorrichtung nach einem der vorhergehenden Ansprüche, **dadurch gekennzeichnet, dass** das optische Element (34; 36) eine Gitterstruktur hat.

7. Vorrichtung nach Anspruch 3, **dadurch gekennzeichnet, dass** das diffraktive optische Element (34; 36) ein Punktmuster erzeugt, insbesondere ein matrixförmiges Punktmuster.

8. Vorrichtung nach einem der vorhergehenden Ansprüche, **dadurch gekennzeichnet, dass** die Strahlungsquelle (12) ein fs-Laser ist.

9. Vorrichtung nach einem der vorhergehenden Ansprüche mit Mitteln zum Einstellen der Abbildung des optischen Systems (10) in Abhängigkeit von der Auswertung des Rechners.

10. Verfahren zum Detektieren der Fokuslage eines optischen Systems (10) unmittelbar vor der Materialbearbeitung, bei dem die Strahlung einer Strahlungsquelle (12) über ein fokussierendes Abbildungssystem (16) in eine Fokusebene (18) abgebildet wird und wobei zur Feststellung der Fokuslage eines optischen Systems unter Einschluss des abbildenden Systems (16) mittels eines optischen Elements (34; 36) im Strahlengang ein Bild am Fokus (18a) erzeugt wird, welches dort reflektiert wird und mit einer Kamera (24) aufgenommen wird, wobei das genannte optische Element (34; 36) das aufgenommene Bild in Abhängigkeit von der Fokussierung der Strahlung beeinflusst und wobei in Abhängigkeit von der genannten Beeinflussung des Bildes eine Aussage über die Fokuslage der fokussierten Strahlung in Bezug auf einen vorgesehenen Fokusort (18a) abgeleitet wird, **dadurch gekennzeichnet, dass** mittels des optischen Elementes (34; 36) die Phase oder Amplitude des Bildes in Abhängigkeit von der Fokuslage beeinflusst wird und dass das optische System eine LASIK-Anordnung ist, wobei die teilreflektierende Fläche (18) einen Prozentsatz der einfallenden Strahlung zur Gewinnung des mit dem digitalen Sensorsystem aufzunehmenden Bildes reflektiert.

11. Verfahren nach Anspruch 10, wobei mittels der genannten abgeleiteten Aussage über die Fokuslage ein optisches Element des optischen Systems (10) zur Änderung der Fokuslage eingestellt wird.

## Claims

1. Apparatus for detecting the focus position of an optical system (10) of a LASIK-arrangement, comprising a radiation source (12), a focussing imaging system (16), a at least partially reflecting surface (18) formed at the rear side of a transparent plate, a digital sensor system (24) for taking a picture reflected at said surface (18) a computer (C) for processing the picture taken by the digital sensor system (24), and an optical element (34; 36) located in the beam path of the optical system (10) before the focusing imaging system (16), **characterized in that** the optical element (34; 36) in the beam path affects the phase or amplitude of said picture in dependency from the focus position, wherein the partially reflective surface (18) reflects a percentage of the impinging radiation to obtain the picture to be taken by the digital sensor system.

2. Apparatus according to claim 1, **characterized in that** the optical element (34; 36) is a matrix of pin holes.

3. Apparatus according to claim 1, **characterized in that** the optical element (34; 36) is a diffractive optical element.

4. Apparatus according to one of the preceding claims, **characterized in that** the optical element (34) is arranged in the beam path of the reflected image.

5. Apparatus according to one of the claim 1 to 3, **characterized in that** the optical element (36) is arranged offset of the beam path of the reflected image.

6. Apparatus according to one the preceding claims, **characterized in that** the optical element (34; 36) has a grid structure.

7. Apparatus according to claim 3, **characterized in that** the diffractive optical element (34; 36) generates a spot pattern, in particular a spot pattern of matrix shape.

8. Apparatus according to one of the preceding claims, **characterized in that** the radiation source (12) is a fs-laser.

9. Apparatus according to one of the preceding claims, comprising means for adjusting the image of the optical system (10) in dependency from the processing of the computer.

10. Method for detecting the focus position of an optical system (10) directly before a material is processed, said method comprising the step of imaging the radiation of a radiation source (12) via a focusing imaging system (16) in a focus plane (18) wherein, for determining the focus position of an optical system including the imaging system (16) by means of an optical element (34; 36) in the beam path, an image is generated at the focus (18a) which is there reflected and taken by a camera (24), wherein said optical element (34; 36) affects the taken picture in dependency from the focusing of the radiation and wherein, in dependency from said affect on the picture, information is derived regarding the focus position of the focused radiation with regard to an intended focus position (18a), **characterized in that**, by means of the optical element (34; 36), the phase or the amplitude of the image is affected in dependency from the focus position and that the optical system is a LASIK-arrangement, wherein the partially reflective surface (18) reflects a percentage of the impinging radiation to obtain the image to be taken by the digital sensor system.

11. Method according to claim 10, wherein, by means of said derived information regarding the focus position, an optical element of the optical system (10) is adjusted to change the focus position.

## Revendications

1. Dispositif de détection de la position focale d'un système optique (10) d'un dispositif de traitement par Lasik, comprenant une source de rayonnement (12), un système de représentation focalisant (16), une surface pour le moins partiellement réfléchissante (18) réalisée à l'arrière d'une plaquette transparente d'un dispositif d'aspiration, un système de détection numérique (24) pour enregistrer l'image réfléchie par ladite surface (18), un ordinateur (C) pour évaluer l'image enregistrée par ledit système de détection numérique (24), et comprenant un élément optique (34 ; 36) placé sur le trajet des rayons du système optique (10) et devant le système de représentation focalisant (16), **caractérisé en ce que** l'élément optique (34 ; 36) placé sur le trajet des rayons influence la phase ou l'amplitude de ladite image en fonction de ladite position focale, la surface partiellement réfléchissante (18) réfléchissant un pourcentage du rayonnement incident pour produire l'image à enregistrer par ledit système de détection numérique.

2. Dispositif selon la revendication 1, **caractérisé en ce que** l'élément optique (34 ; 36) est une matrice perforée.

3. Dispositif selon la revendication 1, **caractérisé en ce que** l'élément optique (34 ; 36) est un élément optique diffractif.

4. Dispositif selon l'une des revendications précédentes, **caractérisé en ce que** l'élément optique (34) est disposé sur le trajet des rayons de ladite image réfléchie.

5. Dispositif selon l'une des revendications 1 à 3, **caractérisé en ce que** l'élément optique (36) est disposé en dehors du trajet des rayons de l'image réfléchie.

6. Dispositif selon l'une des revendications précédentes, **caractérisé en ce que** l'élément optique (34 ; 36) présente une structure en grille.

7. Dispositif selon la revendication 3, **caractérisé en ce que** l'élément optique diffractif (34 ; 36) produit un modèle de projection en points, en particulier un modèle de projection en points sous forme de matrice.

8. Dispositif selon l'une des revendications précédentes, **caractérisé en ce que** la source de rayonnement (12) est un laser femtoseconde.

9. Dispositif selon l'une des revendications précédentes, comprenant des moyens permettant le réglage de la représentation du système optique (10) en fonction de l'évaluation effectuée par l'ordinateur.

10. Procédé de détection de la position focale d'un système optique (10) juste avant le traitement ophthalmologique, dans le cadre duquel le rayonnement émis par une source de rayonnement (12) est représenté dans un plan focal (18) par le biais d'un système de représentation focalisant (16), et dans le cadre duquel, pour déterminer la position focale d'un système optique intégrant ledit système de représentation (16), une image est générée au point de focalisation à l'aide d'un élément optique (34 ; 36) placé sur le trajet des rayons, laquelle image y est réfléchie et enregistrée à l'aide d'une caméra (24), ledit élément optique (34 ; 36) influençant l'image enregistrée en fonction de la focalisation du rayonnement, et une déclaration sur la position focale du rayonnement focalisé par rapport à un lieu focal (18a) prévu étant déduite en fonction de l'influence opérée sur l'image, **caractérisé en ce que** la phase ou l'amplitude de l'image est influencée au moyen de l'élément optique (34 ; 36) en fonction de la position du foyer et **en ce que** le système optique est un dispositif de traitement par Lasik, la surface partiellement réfléchissante (18) réfléchissant un pourcentage du rayonnement incident pour produire l'image à enregistrer par le système de détection numérique.

11. Dispositif selon la revendication 10, ladite déclaration sur la position du foyer établie par déduction permettant le réglage d'un élément optique du système optique (10) en vue de modifier la position du foyer.
